(19) Europäisches Patentamt
European Patent Office
Office européen des brevets

(11) Publication number: **0 034 374**
**B1**

(12) **EUROPEAN PATENT SPECIFICATION**

(45) Date of publication of patent specification: **03.08.83**

(51) Int. Cl.³: **C 07 C 31/02,** C 07 C 31/18, C 07 C 43/13, C 07 C 29/36, C 07 C 41/28

(21) Application number: **81101154.3**

(22) Date of filing: **18.02.81**

(54) **Selective homologation of acetals or ethers to monohydric or polyhydric alcohols.**

(30) Priority: **19.02.80 US 122500**

(43) Date of publication of application:
**26.08.81 Bulletin 81/34**

(45) Publication of the grant of the patent:
**03.08.83 Bulletin 83/31**

(84) Designated Contracting States:
**BE DE FR GB IT NL SE**

(56) References cited:
CA - A - 552 803
US - A - 2 429 878
US - A - 4 062 898
US - A - 4 071 568
US - A - 4 133 966

(73) Proprietor: **UNION CARBIDE CORPORATION**
**Law Department - E134 Old Ridgebury Road**
**Danbury Connecticut 06817 (US)**

(72) Inventor: **Fiato, Rocco Anthony**
**717 Colony Drive**
**Charleston West Virginia 25314 (US)**

(74) Representative: **Schmied-Kowarzik, Volker, Dr. et al,**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 034 374 B1

# Selective homologation of acetals or ethers to monohydric or polyhydric alcohols

Recently, as the price of petroleum has been increasing and as its availability has been becoming more uncertain, considerable interest has risen to alternate means of producing petroleum derivatives, such as monohydric and polyhydric alcohols. One such process is the homologation of acetals or ethers with hydrogen and carbon monoxide. Although these reactions can proceed uncatalyzed, a catalyst is generally employed for the reactions to have commercial viability. A suitable catalyst system containing cobalt, ruthenium and iodine is disclosed in US—A—4,062,898. However, acetal or ether homologation reactions catslyzed by this catalyst system display poor selectivity when the desired products are polyhydric alcohols or their ethers and there is also experienced catalyst instability when the homologation is run at hydrogen to carbon monoxide mole ratios of 2:1 or higher. It is desirable to carry out the homologation with hydrogen to carbon monoxide mole ratios of 2:1 or higher because selectivity to the desired mono- and polyhydric alcohols is improved. A process employing a catalyst system which possesses improved stability at the higher hydrogen to carbon monoxide mole ratios while effectively catalyzing the homologation and improving the selectivity of the homologation to monohydric and polyhydric alcohols would be highly desirable.

US—A—4,071,568 relates to a process for producing monoethers of glycols by the reaction of carbon monoxide and hydrogen with (1) acetals or (2) mixtures of aldehydes and alcohols. The catalyst used is a cobalt compound and at least one other compound which is a trivalent phosphorus compound or a bidentate chelating ligand containing either nitrogen or oxygen. The process disclosed in US—A—4,133,966 is the homologation of methanol by catalytic reaction of methanol with carbon monoxide and hydrogen, with a catalyst system comprising cobalt acetylacetonate, a tertiary organo Group V. A compound of the periodic Table, a first promoter comprising an iodine compound and a second promoter comprising a ruthenium compound.

It has now been found that a catalyst system comprising cobalt, ruthenium, an iodine promoter and an organic phosphine ligand will effectively catalyze the homologation of acetals or ethers and improve the selectivity of the homologation to, monohydric and polyhydric alcohols while retaining, to a large extent, its stability. The homologation reaction can be carried out with or without an inert nonpolar cosolvent.

The process of this invention comprises the homologation of acetals or ethers with hydrogen and carbon monoxide in contact with a catalytically effective amount, sufficient to catalyze the homologation, of a catalyst system containing cobalt, ruthenium, iodine and an organic phosphine ligand to produce mono- and polyhydric alcohols or their ethers having one more carbon atom than the starting acetal or ether.

The acetals which are useful in the process of this invention are the acetals of the formula:

$$\begin{array}{c} R^2 \\ | \\ R^1—O—CH—O—R^3 \end{array}$$

wherein $R^2$ is hydrogen or alkyl of from 1 to 20 carbon atoms, preferably 1 to 4 carbon atoms and the $R^1$ and $R^3$ groups are similar or dissimilar and are alkyl linear or branched, saturated or unsaturated, having from 1 to 20 carbon atoms, preferably from 1 to 4 carbon atoms. It is preferred that $R^1$ and $R^3$ be similar. Illustrative of such acetals one can name methylal, acetaldehyde dimethyl acetal, propionaldehyde dimethyl acetal, formaldehyde diethyl acetal, acetaldehyde diethyl acetal, propionaldehyde diethyl acetal, formaldehyde ethyl methyl acetal, acetaldehyde ethyl methyl acetal, propionaldehyde methyl ethyl acetal and the like. A particularly preferred acetal as a starting material is methylal.

The ethers which are useful in the process of this invention are the ethers of the formula:

$$R^4—O—R^5$$

wherein the $R^4$ and $R^5$ groups are similar or dissimilar and are alkyl, linear or branched, saturated or unsaturated, substituted or unsubstituted having from 1 to 20 carbon atoms, preferably from 1 to 4 carbon atoms wherein the substituents can be hydroxy, alkoxy (preferably with 1 to 4 carbon atoms) or phenoxy or wherein the R groups can be bonded to one another to form a cyclic ether having from 2 to 10 carbon atoms. Illustrative of such ethers one can name dimethyl ether, methyl ethyl ether, diethyl ether, ethyl propyl ether, methyl propyl ether, dipropyl ether, dibutyl ether, ethylene glycol monomethyl ether, ethylene glycol monoethyl ether, 1,3-propanediol monoethyl ether, 1,3-propanediol monomethyl ether, 1,4-butanediol monomethyl ether, 1,4-butanediol monoethyl ether, ethylene oxide oxetane, tetrahydrofuran and the like.

The catalyst system useful in the process of this invention must contain the four components, cobalt, ruthenium, and iodine promoter and an organic phosphine ligand. This four component catalyst system effectively catalyzes the homologation of acetals or ethers with hydrogen and carbon monoxide to selectively produce monohydric and polyhydric alcohols of their ethers.

The cobalt component of the catalyst of this invention can come from a number of sources such as any of the known cobalt carboxylates such as cobalt formate, cobalt acetate, cobalt propionate, cobalt butyrate, cobalt valerate, cobalt hexanoate, and the like; the known cobalt carbonyl compounds such as dicobalt octacarbonyl, methyl cobalt tetracarbonyl, acetyl cobalt tetracarbonyl and the like or their phosphine substituted analogs, many of which are known to those skilled in the art; cobalt oxide and cobalt hydroxide; cobalt carbonate and cobalt bicarbonate; and the soluble cobalt halides such as cobalt iodide, cobalt bromide and cobalt chloride. A convenient source of cobalt is cobalt acetate.

The ruthenium component of the catalyst useful in the process of this invention can come from any source which is capable of providing soluble ruthenium atoms in the reaction. Illustrative of such ruthenium compounds one can name ruthenium trichloride, ruthenium tribromide, ruthenium triiodide, ruthenium acetate, ruthenium acetylacetonate, ruthenium propionate ruthenium octanoate, ruthenium dioxide, ruthenium tetroxide, ruthenium pentacarbonyl, triruthenium dodecacarbonyl and the like. A convenient source of ruthenium is ruthenium trichloride.

Although many soluble halides may be used as a promoter in the catalyst useful in the process of this invention it is preferred that iodine or its derivatives be so employed. Illustrative as sources of the iodide are elemental iodine; cobalt iodide, hydrogen iodide; the alkyl iodides having from 1 to 10 carbon atoms such as methyl iodide, propyl iodide, 2-ethylhexyl iodide, n-decyl iodide and the like; the organic ammonium iodides of the formula $R_4NJ$ wherein R is alkyl having from 1 to 10 carbon atoms such as tetramethyl ammonium iodide and the like, or wherein R is aryl having from 6 to 10 ring carbon atoms such as tetraphenyl ammonium iodide, tetranaphthyl ammonium iodide, tetratolyl ammonium iodide, tetraxylyl ammonium iodide and the like; $R_4PJ$ wherein R is as defined above such as tetramethyl phosphonium iodide, tetrapropyl phosphonium iodide, tetraethylhexyl phosphonium iodide, tetraphenyl phosphonium iodide, tetranaphthyl phosphonium iodide and the like. The preferred source of iodide is elemental iodine.

The organic phosphines which are employed in the catalyst useful in the process of this invention can be phosphines of the formula:

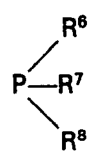

wherein $R^6$, $R^7$, or $R^8$ are individually monovalent organic radicals which either can be dissimilar or any two can be similar or all three can be similar or any two taken together can form an organic divalent cyclic ring system.

The $R^6$, $R^7$ or $R^8$ groups are alkyl, saturated or unsaturated, linear or branched having from 1 to 20 carbon atoms, preferably from 4 to 10 carbon atoms; or aryl, aralkyl or alkaryl having from 6 to 10 ring carbon atoms preferably 6 ring carbon atoms; or cycloalkyl having from 5 to 8 ring carbon atoms, preferably 6 ring carbon atoms. Illustrative of phosphines suitable for use in the catalyst system one can name triethylphosphine, tributylphosphine, triphenylphosphine, tri(4-methoxyphenyl)phosphine, tris(4-tolyl)phosphine, tris(3-chlorophenyl)-phosphine, diphenylhexylphosphine, dimethyl(3-methoxyphenyl)phosphine, dibutylstearyl phosphine, tribenzylphosphine, cyclohexyldibutylphosphine, tricyclohexylphosphine, and the like. Two convenient phosphines are tricyclohexylphosphine and triphenylphosphine.

The R groups may be unsubstituted or they may be substituted with oxygen, sulfur or nitrogen containing groups which do not unduly interfere with the reaction.

The organic phosphines which are employed in the catalyst useful in the process of this invention can also be of the formula:

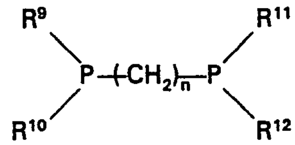

wherein the $R^9$, $R^{10}$, $R^{11}$ and $R^{12}$ groups are similar or dissimilar and are alkyl, linear or branched, substituted or unsubstituted having from 1 to 6 carbon atoms or aryl having from 6 to 10 carbon atoms, and wherein n is an integer having a value of from 2 to 6. Illustrative of such phosphines one can name bis-(dimethylphosphino)ethane, bis-(diethylphosphino)-ethane, bis-(dimethylphosphino)propane, bis-(dimethylphosphino)butane, bis-(dimethylphosphino)hexane, bis-(dipropylphosphino)-ethane, bis-(dihexylphosphino)ethane, bis-(diphenylphosphino)ethane, bis-(diphenylphosphino)propane, bis-(diphenylphosphino)-hexane, bis-(dinaphthylphosphino)ethane, bis-(dinaphthylphosphino)butane, bis-(dimethylphosphino)pentane and the like.

The mole ratio of hydrogen to carbon monoxide may be from 5:1 to 1:5, preferably from 2:1 to 3:1; generally the higher the mole ratio the greater is the selectivity of the reaction to mono- and polyhydric alcohols.

The mole ratio of cobalt to acetal or ether can be from 1:5 to 1:50,000, preferably from 1:25 to 1:400.

The mole ratio of cobalt to ruthenium can be from 1:0.003 to 1:3, preferably from 1:0.03 to 1:0.3.

The mole ratio of cobalt to halide can be from 1:0.1 to 1:25, preferably from 1:1 to 1:5.

The mole ratio of cobalt to organic phosphine

can be from 1:0.1 to 1:100, preferably from 1:0.5 to 1:5.

The mole ratio of phosphine to halide can be from 1:0.001 to 1:250, preferably from 1:0.36 to 1:10. At relative molar concentrations of phosphine to halide below this range the catalyst may exhibit some instability and at relative molar concentrations above this range the catalyst activity may be reduced.

The reaction can be run at a temperature of from 50°C to 250°C, preferably from 100°C to 200°C.

The reaction is run at a pressure of from 70 to 1050 bar (1000 psig to 15,000 psig), preferably from 140 to 420 bar (2000 psig to 6000 psig.)

The time of the reaction will vary and is somewhat dependent on the reaction parameters employed and the individual reactants used.

There can also be present in the reaction mixture from 10 to 90 volume percent, preferably from 40 to 60 volume percent, based on the amount of acetal or ether present, of an inert nonpolar cosolvent such as octane, nonane, dodecane, benzene, toluene, xylene and the like. The presence of the inert nonpolar cosolvent increases the selectivity of the reaction to linear alcohols, vicinal diols and their ethers. While applicant does not intend to be bound by any theory, it is believed that the presence of the inert nonpolar solvent in the amounts indicated increases the selectivity of the reaction to linear alcohols, vicinal diols and their ethers by providing an aprotic nonpolar media for the homologation catalyst thereby allowing polar products and by-products, such as alcohols and water, to be rejected into the polar noncatalyst-containing phase; this minimizes the number and the magnitude of secondary reactions which can occur between these polar, i.e. hydroxylic, materials and the homologation intermediates in the homologation reaction sequence and results in diminished yields of such byproducts as carboxylic acids and esters and increased yields of the desired mono- and polyhydric alcohols. The use of the inert nonpolar cosolvent is also advantageous due to the solubility of the acetal or ether feedstock and the catalyst system in the inert nonpolar solvent and the relative immiscibility of many of these solvents with the product alcohols. This enhances the ease of separation of product from the reaction solution.

In a typical embodiment of a laboratory scale batch process, an acetal or ether is charged to a reactor along with an inert nonpolar solvent and with a catalyst containing a cobalt compound, an iodine compound, a ruthenium compound and a phosphine ligand; the reactor is purged, charged with a hydrogen/carbon monoxide gas mixture, sealed and heated until the desired reaction is completed. It is well known that commercially this process could be run continuously.

The process of this invention offers considerable advantages over the heretofore known processes for homologation of acetals or ethers. By the use of the novel process of this invention, practitioners of the art or acetal or ether homologation can increase the selectivity of the reaction to and hence increase the yield of, the desired mono- and polydric alcohols produced from the acetals or ethers while at the same time maintaining, to a large extent, the stability of the catalyst. This combination of increased yield of desired product and enhanced stability of the homologation catalyst has never been achieved in the acetal or other homologation art; these advantageous results were unexpected and could not have been predicted.

A further advantage provided by the novel process of this invention is that due to the enhanced stability of the catalyst system the reaction can be carried out at lower pressures that have been heretofore required although the reaction temperature is maintained at the levels required for economically viable producton rates. As the catalyst becomes less stable, higher pressures are required to keep the catalyst in a homogenous state; the increased stability of the catalyst of the process of this invention reduces the requirement for the higher pressures. This reduces both the capital equipment costs and the operating costs of producing the desired products and is further evidence of the great utility of the novel process of the invention.

The process described in US—A—4,071,568 does not disclose a suggest the presence of ruthenium in the catalyst. In the instant invention ruthenium is a necessary component of the catalyst system. Since the claimed process is directed to the reaction of an acetal or ether with carbon monoxide and hydrogen to produce monohydric or polyhydric compounds, no relation can be seen to the process disclosed in US—A—4,133,966 which refers to the homologation of methanol to ethanol by reaction with hydrogen and carbon monoxide in the presence of catalysts.

The following examples serve to further illustrate the novel homologation process of this invention.

In these examples and in the experiment which also follows, conversion percent and selectivity percent were determined from vapor phase chromatographic peak area analysis.

## Example 1

In this example the following procedure was used in each run. A glass lined 500 ml autoclave was charged with 50 ml of methylal, 1.2 grams of cobalt acetate tetrahydrate, 0.1 gram of ruthenium trichloride hydrate and elemental iodine and phosphine ligand in the amounts indicated in Table I. In runs 1—5 the phosphine ligand was tricyclohexyl phosphine; in runs 6—9 the phosphine ligand was triphenyl

phosphine. In run 3—5 and 8—9 the methylal charge was 25 ml and each of these runs also contained 25 ml of octane as cosolvent which was included with the initial methylal charge. The autoclave was then sealed and purged with carbon monoxide. The reactor was pressurized to 210 bar (3000 psig) with a gaseous mixture having a 2:1 molar ratio of hydrogen to carbon monoxide, sealed, and the reactor and its contents heated at the average temperature (±3°C) and for the time period reported in Table I. During the reaction the reactor was rocked to obtain thorough mixing. After the indicated reaction time, the reactor was cooled, vented and the liquid contents were isolated. The reaction mixture was analyzed using a vapor phase gas chromatograph equipped with a thermal conductivity detector and a 3,2 mm by 1,8 m column packed with 10 weight percent of polyethylene glycol, having an average molecular weight of about 20,000, on diatomaceous earth, comparing results from standard solutions. In those runs where some of the catalyst precipitated out as metal, the precipitated metal catalyst was washed with methanol, dried and analyzed gravimetrically. The catalyst recovery figures reported in Table I represented the relative amounts of cobalt that remained in solution at the end of the reaction period.

For comparative purposes the above-described procedure was repeated except the phosphine ligand was not included in the catalyst system. In this control run the catalyst system corresponded to that disclosed in US—A—4,062,898. This run contained no octane cosolvent. The results of this run are also shown in Table I.

TABLE I

| Run # | Temp (°C) | Time (hr) | $J_2$ (mmol) | Phosphine Ligand (mmol) | % Selectivity $C_2H_5$ | % Selectivity $CH_3OCH_2CH_2OH$ | % Catalyst Recovery |
|---|---|---|---|---|---|---|---|
| 1 | 175 | 2 | 2 | 4 | 16.7 | 17.1 | 79 |
| 2 | 175 | 2 | 2 | 8 | 13.9 | 20.8 | 89 |
| 3 | 175 | 2 | 2 | 8 | 13 | 28.1 | 100 |
| 4 | 175 | 2 | 0.4 | 8 | 23 | 7.3 | 75 |
| 5 | 150 | 4 | 2 | 8 | 9.2 | 25.8 | 100 |
| 6 | 175 | 2 | 2 | 12 | 13.2 | 20.2 | 100 |
| *7 | 175 | 2 | 2 | 12 | 13.2 | 17 | 100 |
| 8 | 175 | 2 | 2 | 12 | 9 | 21.8 | 100 |
| 9 | 150–175 | 2 | 2 | 12 | 8.6 | 22.8 | 100 |
| Control | 175 | 2 | 2 | 0 | 14.3 | 14.8 | 68 |

* Ruthenium trichloride hydrate charged was 0.05 gram.

As is shown in Table I, the novel process of this invention effectively catalyzes the homologation of methylal and improves the selectivity of the reaction to ethanol and beta-methoxy ethanol over that achievable by a heretofore known catalyst while at the same time displaying significantly improved stability over that shown by the known catalyst. In run 4, the catalyst stability was somewhat reduced due to the higher than preferable phosphine to halide mole ratio that was employed. In all runs, there was 100 percent methylal conversion. Other reaction products formed included methanol, acetaldehyde, methyl acetate and propanol. Comparison of the runs with solvent with those run without solvent indicates that the present of the inert nonpolar cosolvent has a beneficial effect on the selectivity to desired products while also aiding in catalyst stability. This is especially evident when comparing run 3 with run 2; these runs were carried out under completely identical conditions except for the presence of the cosolvent.

## Example 2

To the reactor described in Example 1 there were charged 37.64 grams (39 ml) of beta-methoxy ethanol, 10 grams of water, 1.2 grams of cobalt acetate tetrahydrate 0.1 gram of rithenium trichloride hydrate, 2 grams of

elemental iodine and 2.2 grams of tricyclohexyl phosphine. A procedure similar to that described in Example 1 was foowed; the pressure was 210 bar (3000 psig), the hydrogen to carbon monoxide mole ratio was 2:1, the reaction temperature was 190°C and the reaction time was 2 hours. An analysis, using the procedures described in Example 1, of the reaction products revealed an ether conversion of 73.6 percent and an ethanol selectivity of 18 percent; there was 100 percent catalyst recovery. Other reaction products included ethylene glycol, methanol, propanediol and butanediol. This example demonstrates that the process of this invention effectively converts ethers to monohydric and higher polyhydric alcohols while maintaining excellent catalyst stability at high conversion levels.

Example 3

The apparatus and procedure of Example 2 were repeated except that the beta-methoxy ethanol charge was only 19.3 grams (20 ml), the water charge was only 5 grams and there was additionally charged 17.58 grams (25 ml) of octane as cosolvent. The reaction time was 4 hours; the catalyst system and all other reaction parameters were the same as in Example 2. Analysis, as in Example 2, revealed an ether conversion of 23.7 percent and an ethanol selectivity of 26 percent. This example demonstrates that the presence of the inert nonpolar cosolvent has a beneficial effect on the selectivity of the reaction to desired product. The selectivity to ethanol showed an increase of 44 percent in this example over that attained in Example 2 which was carried out without a cosolvent. The catalyst in this example again demonstrated excellent stability; the catalyst recvery was 100 percent.

Comparative Experiment A

For comparative purposes an ester was used as a starting material with the process of this invention. To the reactor described in Example 1 there was added 50 ml of methyl acetate and the catalyst system and amounts similar to that used in Example 2. The reaction was carried out using the procedures and conditions used in Example 2 except that the reaction temperature was 180°C. An analysis similar to that of Example 2 revealed an ester conversion of only 13.36 percent and an ethanol selectivity of only 8.16 percent; there was no beta-methoxy ethanol formed. This comparative experiment demonstrates that not all methoxy containing compounds are suitable as starting materials for the improved process of this invention.

**Claims**

1. In a process for the production of monohydric and polyhydric alcohols by the reaction of hydrogen carbon monoxide and either an acetal of the formula

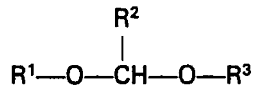

wherein $R^2$ is hydrogen or alkyl of from 1 to 20 carbon atoms, preferably 1 to 4 carbon atoms and the $R^1$ and $R^3$ groups are similar or dissimilar alkyl, linear or branched, saturated or unsaturated, having from 1 to 20 carbon atoms, or an ether of the formula

$$R^4—O—R^5$$

wherein the $R^4$ and $R^5$ groups are similar or dissimilar alkyl, linear or branched, saturated or unsaturated, substituted or unsubstituted, having from 1 to 20 carbon atoms, or wherein the R groups can be bonded to one another to form a cyclic ether having from 2 to 10 carbon atoms; in contact with a catalyst system comprising cobalt, ruthenium and iodine; said process carried out at a temperature of from 50°C to 250°C, and a pressure of from 70 to 1050 bar (1000 psig to 15,000 psig), with a hydrogen to carbon monoxide mole ratio of from 5:1 to 1:5 and with a mole ratio of cobalt to acetal or ether of from 1:5 to 1:50,000; characterized by using with said catalyst system a phosphine ligand of the formula

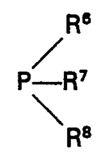

wherein $R^6$, $R^7$, and $R^8$ are individually monovalent organic radicals which are similar or dissimilar, substituted or unsubstituted, or any two taken together can form an organic divalent cyclic ring system, and are alkyl, saturated or unsaturated, linear or branched, having from 1 to 20 carbon atoms, aryl, aralkyl or alkaryl, having from 6 to 10 ring carbon atoms; or cycloalkyl having from 5 to 8 ring carbon atoms; or of the formula

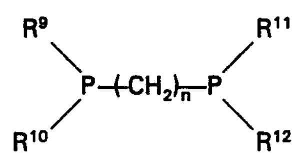

wherein the $R^9$, $R^{10}$, $R^{11}$ and $R^{12}$ groups are similar or dissimilar alkyl, linear or branched, substituted or unsubstituted having from 1 to 6 carbon atoms or aryl having from 6 to 10 carbon atoms, and wherein n is an integer having a value of from 2 to 6; in a concentration such that the mole ratio of cobalt to phosphine ligand is from 1:0.1 to 1:100.

2. An improved process as claimed in claim 1

wherein said phosphine ligand is tricyclohexylphosphine.

3. An improved process as claimed in claim 1 wherein said phosphine ligand is triphenylphosphine.

4. An improved process as claimed in any of the claims 1 to 3 wherein there is additionally present in the reaction mixture from 10 to 90 volume percent, based on the amount of acetal or ether present, or a substantially inert nonpolar cosolvent.

5. An improved process as claimed in claim 4 wherein said cosolvent is octane.

6. An improved process as claimed in any of the claims 1 to 5 for the production of a mixture of ethanol and beta-methoxy ethanol by the reaction of hydrogen, carbon monoxide and methylal and separation and recovery of the components of said mixture.

7. An improved process as claimed in any of the claims 1 to 6 for the production of ethanol by the reaction of hydrogen, carbon monoxide and beta-methoxy ethanol.

8. An improved process as claimed in any of the claims 1 to 7 wherein the mole ratio of cobalt to phosphine ligand is from 1:0.5 to 1:5.

**Revendications**

1. Procédé de production d'alcools monohydroxyliques et polyhydroxyliques par réaction d'hydrogène, d'oxyde de carbone et ou bien d'un acétal de formule

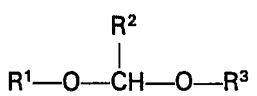

dans laquelle $R^2$ est l'hydrogène ou un radical alkyle ayant 1 à 20 atomes de carbone, de préférence 1 à 4 atomes de carbone, et les groupes $R^1$ et $R^3$ sont des groupes alkyle égaux ou différents, linéaires ou ramifiés, saturés ou non saturés, ayant 1 à 20 atomes de carbone, ou bien d'un éther de formule

$$R^4—O—R^5$$

dans laquelle les groupes $R^4$ et $R^5$ sont des groupes alkyle égaux ou différents, linéaires ou ramifiés, saturés ou non saturés, substitués ou non substitués, ayant 1 à 20 atomes de carbone, ou les groupes R peuvent être liés ensemble pour former un éther cyclique ayant 2 à 10 atomes de carbone; au contact d'un catalyseur comprenant du cobalt, du ruthénium et de l'iode; ledit procédé étant mis en oeuvre à une température de 50 à 250°C et à une pression manométrique de 70 à 1050 bars (1000 à 15 000 lb/in$^2$) avec un rapport molaire de l'hydrogène à l'oxyde de carbone de 5:1 à 1:5 et avec un rapport molaire du cobalt à l'acétal ou à l'éther de 1:5 à 1:50 000; caractérisé par l'utilisation, avec ledit catalyseur, d'un ligand phosphinique de formule

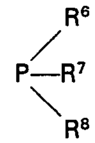

dans laquelle $R^6$, $R^7$ et $R^8$ représentent, individuellement, des radicaux organiques monovalents qui sont égaux ou différents, substitués ou non substitués, ou bien deux quelconques de ces radicaux, pris ensemble, peuvent former un système cyclique organique divalent, et représentent des groupes alkyle, saturés ou non saturés, linéaires ou ramifiés, ayant 1 à 20 atomes de carbone, aryle, aralkyle ou alkaryle ayant 6 à 10 atomes de carbone dans le noyau; ou cycloalkyle ayant 5 à 8 atomes de carbone dans le noyau; ou de formule

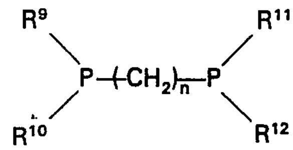

dans laquelle les groupes $R^9$, $R^{10}$, $R^{11}$ et $R^{12}$ sont des groupes alkyle égaux ou différents, linéaires ou ramifiés, substitués ou non substitués ayant 1 à 6 atomes de carbone ou des groupes aryle ayant 6 à 10 atomes de carbone, et *n* est un nombre entier ayant une valeur de 2 à 6; à une concentration choisie de manière que le rapport molaire du cobalt au ligand phosphinique soit de 1:0,1 à 1:100.

2. Procédé perfectionné suivant la revendication 1, dans lequel le ligand phosphinique est la tricyclohexylphosphine.

3. Procédé perfectionné suivant la revendication 1, dans lequel le ligand phosphinique est la triphénylphosphine.

4. Procédé perfectionné suivant l'une quelconque des revendications 1 à 3, dans lequel le mélange réactionnel renferme en outre 10 à 90% en volume, sur la base de la quantité d'acétal ou d'éther présente, d'un cosolvant non polaire pratiquement inerte.

5. Procédé perfectionné suivant la revendication 4, dans lequel le cosolvant est l'octane.

6. Procédé perfectionné suivant l'une quelconque des revendications 1 à 5, pour la production d'un mélange d'éthanol et de bêtaméthoxyéthanol par réaction d'hydrogène, d'oxyde de carbone et de méthylal et séparation et isolement des composants dudit mélange.

7. Procédé perfectionné suivant l'une quelconque des revendications 1 à 6, pour la production d'éthanol par réaction d'hydrogène, d'oxyde de carbone et de bêta-méthoxyéthanol.

8. Procédé perfectionné suivant l'une quelconque des revendications 1 à 7, dans lequel le rapport molaire du cobalt au ligand phosphinique est de 1:0,5 à 1:5.

**Patentansprüche**

1. Verfahren zur Herstellung einwertiger und mehrwertiger Alkohole durch Reaktion von Wasserstoff, Kohlenmonoxid und entweder einem Acetal der Formel

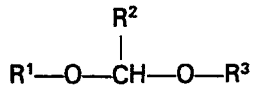

in welcher $R^2$ für Wasserstoff oder Alkyl mit 1 bis 20 Kohlenstoffatomen, vorzugsweise 1 bis 4 Kohlenstoffatomen, steht und die Gruppen $R^1$ und $R^3$ gleiche oder verschiedene, lineare oder verzweigte, gesättigte oder ungesättigte Alkylgruppen mit 1 bis 20 Kohlenstoffatomen sind, oder einem Ether der Formel

$$R^4—O—R^5$$

in welcher die Gruppen $R^4$ und $R^5$ gleiche oder verschiedene, lineare oder verzweigte, gesättigte oder ungesättigte, substituierte oder unsubstituierte Alkylgruppen mit 1 bis 20 Kohlenstoffatomen sind, oder in welcher die Gruppen R unter Bildung eines cyclischen Ethers mit 2 bis 10 Kohlenstoffatomen aneinander gebunden sein können, in Kontakt mit einem Katalysatorsystem, das Kobalt, Ruthenium und Jod enthält, wobei das Verfahren bei einer Temperatur von 50 bis 250°C und einem Druck von 70 bis 1050 bar (1000 psig bis 15 000 psig) mit einem Mol-Verhältnis von Wasserstoff zu Kohlenmonoxid von 5:1 bis 1:5 und mit einem Mol-Verhältnis von Kobalt zu Acetal oder Ether von 1:5 bis 1:50 000 durchgeführt wird, dadurch gekennzeichnet, daß mit dem Katalysatorsystem ein Phosphinligand der Formel

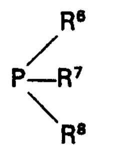

in welcher $R^6$, $R^7$ und $R^8$ einzeln einwertige organische Reste sind, die gleich oder verschieden, substituiert oder unsubstituiert sind, oder von denen zwei beliebige zusammen ein organisches zweiwertiges cyclisches Ringsystem bilden können und für gesättigtes oder ungesättigtes, lineares oder verzweigtes Alkyl mit 1 bis 20 Kohlenstoffatomen, Aryl, Aralkyl oder Alkaryl mit 6 bis 10 Ringkohlen stoffatomen oder Cycloalkyl mit 5 bis 8 Ringkohlenstoffatomen stehen; oder der Formel

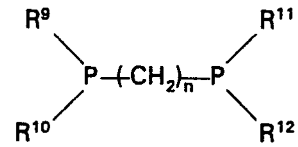

in welcher die Gruppen $R^9$, $R^{10}$, $R^{11}$ und $R^{12}$ gleiche oder verschiedene, lineare oder verzweigte, substituierte oder unsubstituierte Alkylgruppen mit 1 bis 6 Kohlenstoffatomen oder Aryl mit 6 bis 10 Kohlenstoffatomen bedeuten, und in welcher n eine ganze Zahl mit einem Wert von 2 bis 6 ist; in einer solchen Konzentration, daß das Mol-Verhältnis von Kobalt zu Phosphinliganden 1:0,1 bis 1:100 beträgt, verwendet wird.

2. Verbessertes Verfahren gemäß Anspruch 1, in welchem der Phosphinligand Tricyclohexylphosphin ist.

3. Verbessertes Verfahren gemäß Anspruch 1, in welchem der Phosphinligand Triphenylphosphin ist.

4. Verbessertes Verfahren gemäß einem der Ansprüche 1 bis 3, in welchem zusätzlich in der Reaktionmischung 10 bis 90 Vol.-% eines praktisch inerten, nicht-polaren Kolösungsmittels, bezogen auf die anwesende Menge an Acetal oder Ether, anwesend sind.

5. Verbessertes Verfahren gemäß Anspruch 4, in welchem das Kolösungsmittel Octan ist.

6. Verbessertes Verfahren gemäß einem der Ansprüche 1 bis 5 zur Herstellung einer Mischung aus Ethanol und $\beta$-Methoxyethanol durch Reaktion von Wasserstoff, Kohlenmonoxid und Methylal und Trennung und Gewinnung der Komponenten aus der Mischung.

7. Verbessertes Verfahren gemäß einem der Ansprüche 1 bis 6 zur Herstellung von Ethanol durch Reaktion von Wasserstoff, Kohlenmonoxid und $\beta$-Methoxyethanol.

8. Verbessertes Verfahren gemäß einem der Ansprüche 1 bis 7 in welchem das Mol-Verhältnis von Kobalt zu Phosphinliganden 1:0,5 bis 1:5 beträgt.